(19) Europäisches Patentamt
European Patent Office
Office européen des brevets

(11) EP 0 966 251 B1

(12) **FASCICULE DE BREVET EUROPEEN**

(45) Date de publication et mention de la délivrance du brevet:
**14.12.2005 Bulletin 2005/50**

(21) Numéro de dépôt: **98939695.7**

(22) Date de dépôt: **16.07.1998**

(51) Int Cl.[7]: **A61K 7/13**

(86) Numéro de dépôt international:
**PCT/FR1998/001561**

(87) Numéro de publication internationale:
**WO 1999/011229 (11.03.1999 Gazette 1999/10)**

# (54) COMPOSITION POUR LA TEINTURE D'OXYDATION DES FIBRES KERATINIQUES COMPRENANT DU 2-CHLORO 6-METHYL 3-AMINOPHENOL ET DEUX BASES D'OXYDATION, ET PROCEDE DE TEINTURE

OXIDATIONSFÄRBEMITTEL FÜR KERATINFASERN ENTHALTEND EIN 2-CHLOR- 6-METHYL-3-AMINOPHENOL UND ZWEI OXIDATIONSBASEN, SOWIE DAS FÄRBEVERFAHREN

OXIDATION DYEING COMPOSITION FOR KERATIN FIBRES COMPRISING 2-CHLORO 6-METHYL 3-AMINOPHENOL AND TWO OXIDATION BASES, AND DYEING METHOD

(84) Etats contractants désignés:
**AT BE CH DE ES FR GB IT LI NL SE**

(30) Priorité: **01.09.1997 FR 9710855**

(43) Date de publication de la demande:
**29.12.1999 Bulletin 1999/52**

(73) Titulaire: **L'OREAL**
**75008 Paris (FR)**

(72) Inventeur: **AUDOUSSET, Marie-Pascale**
**F-92600 Asnières (FR)**

(74) Mandataire: **Fevrier, Murielle Françoise E.**
**L'OREAL - D.I.P.I.**
**25-29 Quai Aulagnier**
**92600 Asnières (FR)**

(56) Documents cités:
**EP-A- 0 039 030**
**EP-A- 0 063 736**
**EP-A- 0 256 468**
**EP-A- 0 591 059**
**WO-A-90/12562**
**WO-A-94/27564**
**WO-A-96/15765**
**WO-A-96/15766**
**DE-A- 4 122 748**
**DE-A- 4 205 329**
**DE-A- 4 344 551**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 966 251 B1

## Description

La présente invention a pour objet une composition pour la teinture d'oxydation des fibres kératiniques, en particulier des fibres kératiniques humaines telles que les cheveux, comprenant du 2-chloro 6-méthyl 3-aminophénol à titre de coupleur, en association avec au moins deux bases d'oxydation différentes l'une de l'autre, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrimidine, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 016 008 des compositions pour la teinture d'oxydation des fibres kératiniques contenant à titre de coupleur du 2-chloro 6-méthyl 3-aminophénol ou du 2-méthyl 5-chloro 3-aminophénol, en association avec des bases d'oxydation classiquement utilisées pour la teinture d'oxydation, telles que par exemple certaines paraphénylènediamines ou du para-aminophénol. De telles compositions ne sont cependant pas entièrement satisfaisantes notamment du point de vue de la tenue des colorations obtenues vis à vis des diverses agressions que peuvent subir les cheveux et en particulier vis à vis des shampooings et des déformations permanentes.

Il a également été proposé, dans les demandes de brevet WO 96/15765 et WO 96115766, des compositions pour la teinture d'oxydation des fibres kératiniques contenant l'association spécifique du 2-chloro 6-méthyl 3-aminophénol à titre de coupleur et d'une base d'oxydation particulière comme la 2-β-hydroxyéthyl paraphénylènediamine et/ou la tétraaminopyrimidine et certains para-aminophénols tels que par exemple le 3-méthyl 4-aminophénol, le 2-allyl 4-aminophénol ou bien encore le 2-aminométhyl 4-aminophénol. De telles compositions ne sont cependant pas non plus entièrement satisfaisantes notamment du point de vue de la puissance des colorations obtenues.

Or, la demanderesse vient maintenant de découvrir qu'il est possible d'obtenir de nouvelles teintures puissantes et particulièrement résistantes aux diverses agressions que peuvent subir les cheveux, en associant du 2-chloro 6-méthyl 3-aminophénol et au moins deux bases d'oxydation différentes l'une de l'autre.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- du 2-chloro 6-méthyl 3-aminophénol et/ou au moins l'un de ses sels d'addition avec un acide, à titre de coupleur,
- et au moins deux bases d'oxydation différentes l'une de l'autre choisie parmi les para-phénylènediamines, les bases doubles, les para-amonophénols, et les bases d'oxydation hétérocycliques, étant entendu que ladite composition ne renferme pas simultanément de la 2-β-hydroxyéthyl paraphénylènediamine et de la tétraaminopyrimidine.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

Parmi les paraphénylènediamines utilisables à titre de base d'oxydation dans les composition tinctoriales conformes à l'invention, on peut notamment citer les composés de formule (I) suivante et leurs sels d'addition avec un

acide :

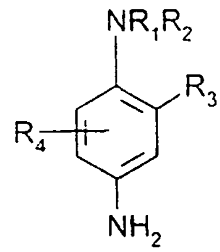

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle

ou 4'-aminophényle ;

- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy(C,-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_4$ représente un atome d'hydrogène d'halogène ou un radical alkyle en $C_1$-$C_4$.

Parmi les groupements azotés de la formule (I) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N, N-(éthyl, p-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl)amino paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide :

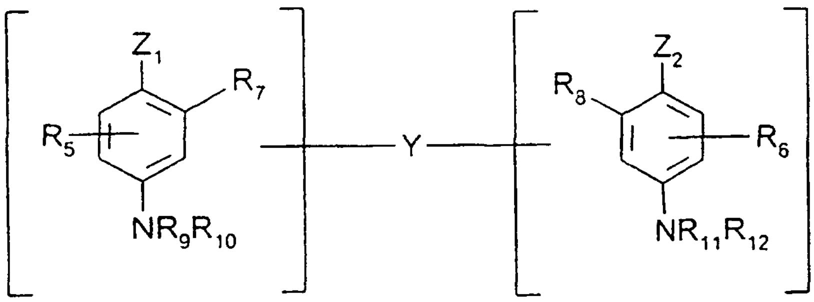

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_1$-$C_4$, aminoalkyle en $C_1$-$C_4$ ou un bras de liaison Y ;
- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide :

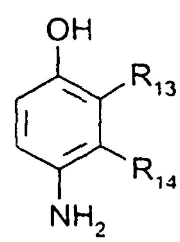

(III)

dans laquelle :

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$,

polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$), étant entendu qu'au moins un des radicaux $R_{13}$ ou $R_{14}$ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-dimainopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino, 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique :

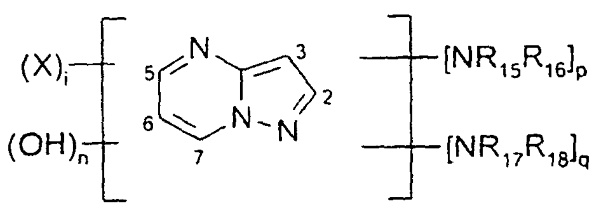

(IV)

dans laquelle :

- $R_{15}$, $R_{16}$, $R_{17}$ et $R_{18}$, identique ou différents désignent, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical ($C_1$-$C_4$)alcoxy alkyle en $C_1$-$C_4$, un radical aminoalkyle en $C_1$-$C_4$ (l'amine pouvant être protégée par un radical acétyle, uréido ou sulfonyle), un radical ($C_1$-$C_4$)alkylamino alkyle en $C_1$-$C_4$, un radical di-[($C_1$-$C_4$)alkyl] amino alkyle en $C_1$-$C_4$ (les radicaux dialkyles pouvant former un cycle carboné ou un hétérocycle à 5 ou 6 chaînons), un radical hydroxy ($C_1$-$C_4$)alkyl- ou di-[hydroxy($C_1$-$C_4$) alkyl]-amino alkyle en $C_1$-$C_4$;

- les radicaux X désignent , identiques ou différents, un atome d'hydrogène, un radical alkyle en $C_1$-$C_4$, un radical aryle, un radical hydroxyalkyle en $C_1$-$C_4$, un radical polyhydroxyalkyle en $C_2$-$C_1$, un radical amino alkyle en $C_1$-$C_4$, un radical ($C_1$-$C_4$)alkyl amino alkyle en $C_1$-$C_4$, un radical di-[($C_1$-$C_4$)alkyl] amino alkyle en $C_1$-$C_4$ (les dialkyles pouvant former un cycle carboné ou un hétérocycle à 5 ou 6 chaînons), un radical hydroxy($C_1$-$C_4$)alkyl ou di-[hydroxy($C_1$-$C_4$ )alkyl]amino alkyle en $C_1$-$C_4$, un radical amino, un radical ($C_1$-$C_4$)alkyl- ou di-[($C_1$-$C_4$)alkyl]-amino ;

un atome d'halogène, un groupe acide carboxylique, un groupe acide sulfonique ;

- i vaut 0, 1, 2 ou 3 ;
- p vaut 0 ou 1 ;
- q vaut 0 ou 1 ;
- n vaut 0 ou 1 ;

sous réserve que :

- la somme p + q est différente de 0 ;
- lorsque p + q est égal à 2, alors n vaut 0 et les groupes $NR_{15}R_{16}$ et $NR_{17}R_{18}$ occupent les positions (2,3) ; (5,6) ; (6,7) ; (3,5) ou (3,7) ;
- lorsque p + q est égal à 1 alors n vaut 1 et le groupe $NR_{15}R_{16}$ (ou $NR_{17}R_{18}$) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en $\alpha$ d'un atome d'azote, il existe un équilibre tautomérique représenté par exemple par le schéma suivant :

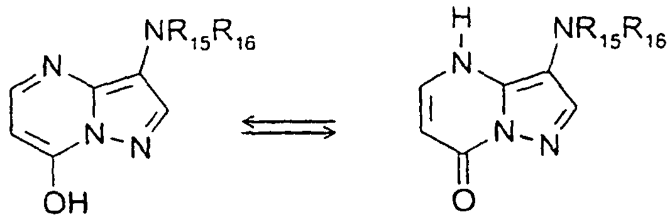

Parmi les pyrazolo-[1,5-a]-pyrimidines de formule (IV) ci-dessus on peut notamment citer :

- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;

et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :

- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977,
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :

- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

Le 2-chloro 6-méthyl 3-aminophénol et/ou le ou ses sels d'addition avec un acide, représentent de préférence de 0,0001 à 5 % en poids environ du poids total de la composition tinctoriale, et encore plus préférentiellement de 0,005 à 3 % en poids environ de ce poids.

L'ensemble des bases d'oxydation conformes à l'invention représente de préférence de 0,0005 à 12 % en poids environ du poids total de la composition tinctoriale, et encore plus préférentiellement de 0,005 à 6 % en poids environ de ce poids.

Les compositions tinctoriales conformes à l'invention peuvent contenir d'autres coupleurs différents du 2-chloro 6-méthyl 3-aminophénol et/ou des colorants directs notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. Les solvants organiques préférés sont les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

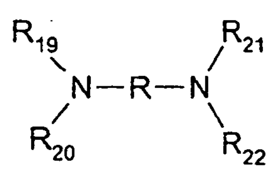

(V)

dans laquelle R est un reste propylène éventuellement substitué par un groupement hydroxyle ou un radical alkyle en $C_1$-$C_4$ ; $R_{19}$, $R_{20}$, $R_{21}$ et $R_{22}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas,

ou substantiellement pas, altérées par la ou les adjonctions envisagées.

La composition tinctoriale selon l'invention peut se présenter sous des formes diverses, telles que sous forme de liquides, de crèmes ou de gels.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

## EXEMPLES

### <u>EXEMPLES DE TEINTURE COMPARATIFS 1 ET 2</u>

On a préparé les compositions tinctoriales, conformes à l'invention, suivantes (teneurs en grammes) :

| EXEMPLE | 1 | 2 (*) |
|---|---|---|
| 2-chloro 6-méthyl 3-aminophénol (coupleur) | 1,182 | 1,182 |
| Paraphénylènediamine (base d'oxydation) | 0,81 | - |
| Sulfate de tétraaminopyrimidine (base d'oxydation) | 0,13 | 0,13 |
| Dichlorhydrate de 2-β-hydroxyéthyl paraphénylènediamine (base d'oxydation) | - | 1,687 |

(*) : exemple ne faisant pas partie de l'invention

(suite)

| EXEMPLE | 1 | 2 (*) |
|---|---|---|
| Support de teinture commun | (**) | (**) |
| Eau déminéralisée q.s.p. | 100 g | 100 g |

(*) : exemple ne faisant pas partie de l'invention

(**) support de teinture commun :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12® par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10 g

Au moment de l'emploi, on a mélangé chaque composition tinctoriale ci-dessus avec une demie quantité en poids d'une composition oxydante constituée par une solution d'eau oxygénée à 10 volumes (3 % en poids).

Chaque composition résultante a été appliquée pendant 30 minutes sur des mèches de cheveux gris naturels à 90 % de blancs et sur des mèches de cheveux gris permanentés à 90 % de blancs. Les mèches de cheveux ont ensuite été rincées, lavées avec un shampooing standard puis séchées.

La couleur de chaque mèche de cheveux teintes avec les compositions 1 et 2 a été évaluée dans le système MUNSELL au moyen d'un colorimètre CM 2002 MINOLTA®.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

Pour chaque composition, la différence entre la couleur de la mèche de cheveux gris naturels la couleur de la mèche de cheveux gris permanentés a été calculée en appliquant la formule de NICKERSON :

$$\Delta E = 0{,}4C_0 dH + 6dV + 3dC$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et C0 représente la pureté de la mèche par rapport à laquelle on désire évaluer la différence de couleur.

La différence de couleur ainsi calculée et exprimée par le ΔE reflète la sélectivité des colorations qui est d'autant plus faible que la valeur du ΔE est basse.

Les résultats sont donnés dans le tableau ci-dessous :

| EXEMPLE | Couleur obtenue sur cheveux naturels | Couleur obtenue sur cheveux permanentés | **Sélectivité de la coloration** | | | |
|---|---|---|---|---|---|---|
| | | | ΔH | ΔV | ΔC | ΔE |
| 1 | 9,4 P 2,1 / 1,6 | 8,1 P 1,9 / 1,3 | 1,3 | 0,2 | 0,3 | 2,9 |
| 2 (*) | 4,2 P 2,6/2,5 | 3,5 P 2,0 / 1,8 | 0,7 | 0,6 | 0,7 | 6,4 |

Ces résultats montrent que la coloration obtenue en mettant en oeuvre la composition tinctoriale conforme à l'invention de l'exemple 1, c'est à dire contenant l'association spécifique du 2-chloro 6-méthyl 3-aminophénol de la paraphénylènediamine et de la tétraaminopyrimidine est nettement moins sélective que la coloration obtenue en mettant en oeuvre la composition de l'exemple 2 ne faisant pas partie de l'invention car contenant l'association du 2-chloro

6-méthyl 3-aminophénol, de la 2-β-hydroxyéthyl paraphénylènediamine et de la tétraaminopyrimidine telle que décrite dans la demande de brevet WO96/15765.

**EXEMPLES 3 à 5 DE TEINTURE**

On a préparé les compositions tinctoriales, conformes à l'invention, suivantes (teneurs en grammes) :

| EXEMPLE | 3 | 4 | 5 |
|---|---|---|---|
| 2-chloro 6-méthyl 3-aminophénol (coupleur) | 0,471 | 0,471 | 0,471 |
| Tétrachlorhydrate de N,N'-bis-(β-hydroxyéthyl) N,N'-bis-(4'-aminophényl) 1,3-diamino propanol (base d'oxydation) | 0,259 | - | - |
| Para-aminophénol (base d'oxydation) | - | 0,163 | - |
| Dichlorhydrate de pyrazolo-[1,5-a]-pyrimidine-3,7-diamine (base d'oxydation) | - | - | 0,166 |
| Paraphénylènediamine (base d'oxydation) | - | 0,162 | - |
| Sulfate de N,N-bis-(β-hydroxyéthyl) paraphénylènediamine (base d'oxydation) | - | - | 0,22 |
| Dichlorhydrate de 4,5-diamino 1-éthyl 3-méthyl pyrazole (base d'oxydation) | 0,319 | - | - |
| Support de teinture commun n°2 | (***) | (***) | - |
| Support de teinture commun n°3 | - | - | (****) |
| Eau déminéralisée q.s.p. | 100 g | 100 g | 100 g |

(***) support de teinture commun n°2 : - Ethanol à 96 ° 18 g
- Métabisulfite de sodium en solution aqueuse à 35 % 0,68 g
- Sel pentasodique de l'acide diéthylènetriaminopentacétique 1,1 g
- Ammoniaque à 20 % de $NH_3$ 10 g

(****) support de teinture commun n°3 : - Ethanol à 96 ° 18 g
- Métabisulfite de sodium en solution aqueuse à 35 % 0,68 g
- Sel pentasodique de l'acide diéthylènetriaminopentacétique 1,1 g
- Tampon $K_2HPO_4$ / $KH_2PO_4$ (1,5M / 1 M) 10 g

Au moment de l'emploi, on a mélangé chaque composition tinctoriale ci-dessus avec une quantité égale en poids d'une composition oxydante constituée par une solution d'eau oxygénée à 20 volumes (6 % en poids).
Chaque composition résultante a été appliquée pendant 30 minutes sur des mèches de cheveux gris naturels à 90 % de blancs. Les mèches de cheveux ont ensuite été rincées, lavées avec un shampooing standard puis séchées.
Les nuances obtenues figurent dans le tableau ci-dessous :

| EXEMPLE | pH de TEINTURE | NUANCE OBTENUE |
|---|---|---|
| 3 | 10 ± 0,2 | Violine cendré |
| 4 | 10 ± 0,2 | Rouge acajou |
| 5 | 6,8 ± 0,2 | Cendré violine |

## Revendications

1. Composition pour la teinture d'oxydation des fibres kératiniques humaines - telles que les cheveux, **caractérisée par le fait qu'**elle comprend, dans un milieu approprié pour la teinture :

   - du 2-chloro 6-méthyl 3-aminophénol et/ou au moins l'un de ses sels d'addition avec un acide, à titre de coupleur,
   - et au moins deux bases d'oxydation différentes choisies parmi les para-phénylènediamines, les bases doubles, les para-aminophénols et les bases d'oxydation hétérocycliques ; étant entendu que ladite composition ne renferme pas simultanément de la 2-β-hydroxyéthyl paraphénylènediamine et de la tétraaminopyrimidine.

2. Composition selon la revendication 1, **caractérisée par le fait que** les paraphénylènediamines sont choisies parmi les composés de formule (I) suivante et leurs sels d'addition avec un acide:

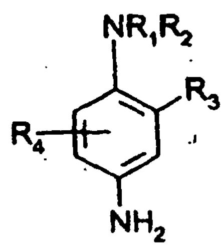

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_4$ représente un atome d'hydrogène d'halogène ou un radical alkyle en $C_1$-$C_4$.

3. Composition selon la revendication 2, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl)amino paraphénylènediamine, et leurs sels d'addition avec un acide.

4. Composition selon l'une quelconque des revendications précédentes, **caractérisée par le fait que** les bases doubles sont choisies parmi les composés répondant à la formule (II) suivante, et leurs sels d'addition avec un acide:

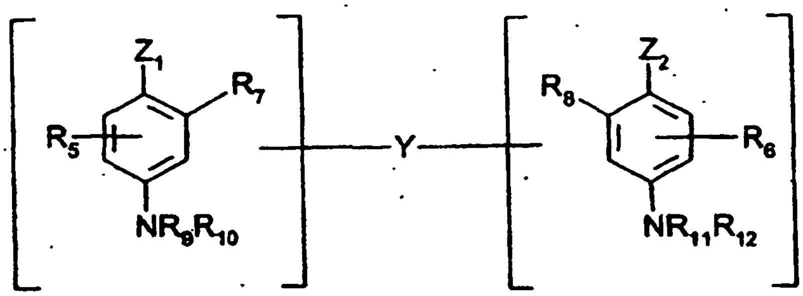

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_8$ ;

- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.

**5.** Composition selon la revendication 4, **caractérisée par le fait que** les bases doubles de formule (II) sont choisies parmi le N,N'-bis-(β-hydroxyéthyl) N,N'-bis-(4'-aminophényl) 1,3-diamino propanol, la N,N'-bis-(β-hydroxyéthyl) N, N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N-bis-(4-méthylaminophényl) tétraméthylènediamine, la N,N'-bis-(éthyl) N,N'-bis-(4'-amino. 3'-méthylphényl) éthylènediamine, le 1,8-bis-(2,5-diaminophénoxy)-3,5-dioxaoctane, et leurs sels d'addition avec un acide.

**6.** Compositions selon l'une quelconque des revendications précédentes, **caractérisée par le fait que** les para-aminophénols sont choisis parmi les composés répondant à la formule (III) suivante, et leurs sels d'addition avec un acide :

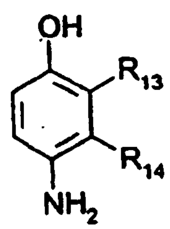

(III)

dans laquelle:

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$), étant entendu qu'au moins un des radicaux $R_{13}$ ou $R_{14}$ représente un atome d'hydrogène.

**7.** Compositions selon la revendication 6, **caractérisée par le fait que** les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

**8.** Composition selon l'une quelconque des revendications précédentes **caractérisée par le fait que** les bases hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

**9.** Composition selon l'une quelconque des revendications précédentes, **caractérisée par le fait que** le 2-chloro 6-méthyl 3-aminophénol et/ou le ou ses sels d'addition avec un acide représente de 0,0001 à 5% en poids du poids total de la composition tinctoriale.

**10.** Composition selon la revendication 9, **caractérisée par le fait que** le 2-chloro 6-méthyl 3-aminophénol et/ou le ou ses sels d'addition avec un acide représente de 0,005 à 3% en poids du poids total de la composition tinctoriale.

**11.** Composition selon l'une quelconque des revendications précédentes, **caractérisée par le fait que** l'ensemble des bases d'oxydation représente de 0,0005 à 12% en poids du poids total de la composition tinctoriale.

**12.** Composition selon la revendication 11, **caractérisée par le fait que** l'ensemble des bases d'oxydation représente de 0,005 à 6% en poids du poids total de la composition tinctoriale.

**13.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**14.** Composition selon l'une quelconque des revendications précédentes, **caractérisée par le fait qu'**elle présente un pH compris entre 3 et12.

**15.** Composition selon l'une quelconque des revendications précédentes, **caractérisée par le fait qu'**elle se présente sous forme de liquides, de crèmes ou de gels.

**16.** Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**17.** Procédé selon la revendication 16, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates, les peracides.

**18.** Dispositif à plusieurs compartiments, ou "kit" de teinture à plusieurs compartiments, dont un premier compartiment renferme une composition tinctoriale telle que définie à l'une "quelconque des revendications 1 à 15 et un second compartiment renferme une composition oxydante.

**Patentansprüche**

**1.** Zusammensetzung zum oxidativen Färben von menschlichen Keratinfasern, wie Haaren, **dadurch gekennzeichnet, dass** sie in einem zum Färben geeigneten Medium enthält:

. das 2-Chlor-6-methyl-3-aminophenol und/oder mindestens eines seiner Additionssalze mit einer Säure als Kuppler;

. mindestens zwei Oxidationsbasen, die voneinander verschieden sind und die unter den p-Phenylendiaminen, Doppelbasen, p-Aminophenolen und den heterocyclischen Oxidationsbasen ausgewählt sind; mit der Maßgabe, dass die Zusammensetzung nicht gleichzeitig das 2-β-Hydroxyethylp-phenylendiamin und das Tetraaminopyrimidin enthält.

**2.** Zusammensetzung nach Anspruch 1, **dadurch gekennzeichnet, dass** die p-Phenylendiamine unter den Verbindungen der folgenden Formel (I) und deren Additionssalzen mit einer Säure ausgewählt sind:

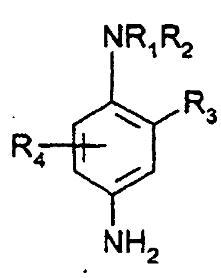

(I)

worin bedeuten:

- $R_1$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, eine mit einer stickstoffhaltigen Gruppe substituierte $C_{1-4}$-Alkylgruppe, Phenyl oder 4'-Aminophenyl,
- $R_2$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl oder eine mit einer stickstoffhaltigen Gruppe substituierte $C_{1-4}$-Alkylgruppe,
- $R_3$ Wasserstoff, Halogen, wie Chlor, Brom, Iod oder Fluor, $C_{1-4}$-Alkyl, $C_{1-4}$-Monohydroxyalkyl, $C_{1-4}$-Hydroxyalkoxy, $C_{1-4}$-Acetylaminoalkoxy, $C_{1-4}$-Mesylaminoalkoxy oder $C_{1-4}$-Carbamoylaminoalkoxy, und
- $R_4$ Wasserstoff, Halogen oder $C_{1-4}$-Alkyl.

**3.** Zusammensetzung nach Anspruch 2, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N, N-Bis(β-hydroxyethyl)-p-phenylendiamin, 4-Amino-N,N-bis(β-hydroxyethyl)-3-methyl-anilin, 4-Amino-3-chlor-N, N-bis(β-hydroxyethyl)-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-pheriylendiamin, N-(β-Methoxyethyl)amino-p-phenylendiamin und deren Additionssalzen mit einer Säure ausgewählt sind.

**4.** Zusammensetzung nach einem der vorhergehenden Ansprüche, **dadurch gekennzeichnet, dass** die Doppelbasen unter den Verbindungen der folgenden Formel (II) und deren Additionssalze mit einer Säure ausgewählt sind:

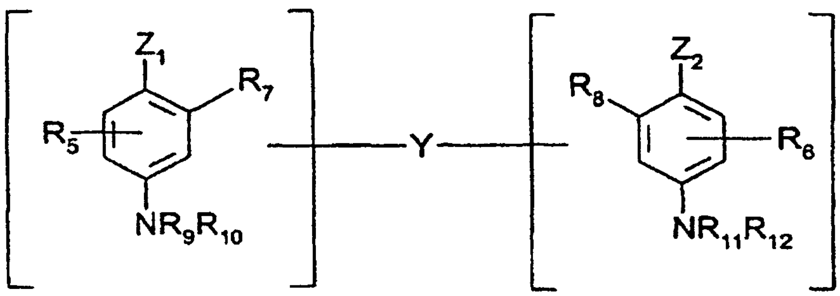

(II)

worin bedeuten:

- $Z_1$ und $Z_2$, die identisch oder voneinander verschieden sind, eine Hydroxygruppe oder eine $NH_2$-Gruppe, die mit $C_{1-4}$-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder $C_{1-6}$-Alkoxygruppen substituiert sein kann,
- $R_5$ und $R_6$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl oder eine Verbindungsgruppe Y,
- $R_7$, $R_8$, $R_9$, $R_{10}$, $R_{11}$ und $R_{12}$, die gleich oder verschieden sind, Wasserstoff, eine Verbindungsgruppe Y oder $C_{1-4}$-Alkyl, mit der Maßgabe, dass die Verbindungen der Formel (II) nur eine Verbindungsgruppe Y pro Molekül aufweisen.

**5.** Zusammensetzung nach Anspruch 4, **dadurch gekennzeichnet, dass** die Doppelbasen der Formel (II) unter N, N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis (4-aminophenyl)-tetramethylendiamin, N,N'-Bis(4-methylaminophenyl)-tetramethylendiamin, N,N'-Bis(ethyl)-N, N'-bis(4'-amino-3'-methylphenyl)-ethylendiamin, 1,8-Bis(2,5-diaminophenoxy)-3,5-dioxaoctan und deren Additionssalzen mit einer Säure ausgewählt sind.

**6.** Zusammensetzung nach einem der vorhergehenden Ansprüche, **dadurch gekennzeichnet, dass** die p-Aminophenole unter den Verbindungen der folgenden Formel (III) und deren Additionssalzen mit einer Säure ausgewählt sind:

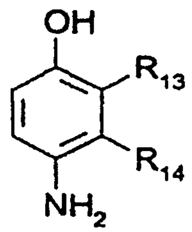

(III)

worin bedeuten:

- $R_{13}$ Wasserstoff, Halogen, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-Hydroxyalkyl-$C_{1-4}$-aminoalkyl,
- $R_{14}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-alkyl,

mit der Maßgabe, dass mindestens eine der Gruppen $R_{13}$ oder $R_{14}$ Wasserstoff bedeutet.

**7.** Zusammensetzung nach Anspruch 6, **dadurch gekennzeichnet, dass** die p-Aminophenole unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

**8.** Zusammensetzung nach einem der vorhergehenden Ansprüche, **dadurch gekennzeichnet, dass** die heterocyclischen Basen unter den Pyridinderivaten, Pyrimidinderivaten, Pyrazolderivaten, Pyrazolo-pyrimidinderivaten und deren Additionssalzen mit einer Säure ausgewählt sind.

**9.** Zusammensetzung nach einem der vorhergehenden Ansprüche, **dadurch gekennzeichnet, dass** das 2-Chlor-6-methyl-3-aminophenol und/oder sein(e) Additionssalz(e) mit einer Säure 0,0001 bis 5 Gew.-% des Gesamtgewichts der Farbmittelzusammensetzung ausmachen.

**10.** Zusammensetzung nach Anspruch 9, **dadurch gekennzeichnet, dass** das 2-Chlor-6-methyl-3-aminophenol und/oder sein(e) Additionssalz(e) mit einer Säure 0,005 bis 3 Gew.-% des Gesamtgewichts der Farbmittelzusammensetzung ausmachen.

**11.** Zusammensetzung nach einem der vorhergehenden Ansprüche, **dadurch gekennzeichnet, dass** die Oxidationsbasen insgesamt 0,0005 bis 12 Gew.-% des Gesamtgewichts der Farbmittelzusammensetzung ausmachen.

**12.** Zusammensetzung nach Anspruch 11, **dadurch gekennzeichnet, dass** die Oxidationsbasen insgesamt 0,005 bis 6 Gew.-% des Gesamtgewichts der Farbmittelzusammensetzung ausmachen.

**13.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**14.** Zusammensetzung nach einem der vorhergehenden Ansprüche, **dadurch gekennzeichnet, dass** sie einen pH-Wert im Bereich von 3 bis 12 aufweist.

**15.** Zusammensetzung nach einem der vorhergehenden Ansprüche, **dadurch gekennzeichnet, dass** sie als Flüssigkeit, Creme oder Gel vorliegt.

**16.** Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche

1 bis 15 aufgebracht wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

17. Verfahren nach Anspruch 16, **dadurch gekennzeichnet, dass** das in der oxidierenden Zusammensetzung vorliegende Oxidationsmittel unter Wasserstoffperoxid, Harnstoffperoxid, Alkalimetallbromaten, Salzen von Persäuren, wie Perboraten und Persulfaten, und Persäuren ausgewählt ist.

18. Vorrichtung mit mehreren Abteilungen oder "Kit" zum Färben mit mehreren Abteilungen, **dadurch gekennzeichnet, dass** eine Abteilung die in den Ansprüchen 1 bis 15 definierte Farbmittelzusammensetzung und eine zweite Abteilung eine oxidierende Zusammensetzung enthält.

## Claims

1. Composition for the oxidation dyeing of human keratinous fibres, such as hair, **characterized in that** it comprises, in a medium appropriate for dyeing:

- 2-chloro-6-methyl-3-aminophenol and/or at least one of its addition salts with an acid, as coupler;
- and at least two different oxidation bases chosen from para-phenylenediamines, double bases, para-aminophenols and heterocyclic oxidation bases;

it being understood that the said composition does not simultaneously include 2-(β-hydroxyethyl)-para-phenylenediamine and tetraaminopyrimidine.

2. Composition according to Claim 1, **characterized in that** the para-phenylenediamines are chosen from the compounds of following formula (I) and their addition salts with an acid:

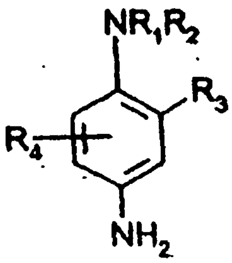

(I)

in which:

- $R_1$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)-alkyl radical, a $C_1$-$C_4$ alkyl radical substituted by a nitrogenous group, a phenyl radical or a 4'-aminophenyl radical;
- $R_2$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)-alkyl radical or a $C_1$-$C_4$ alkyl radical substituted by a nitrogenous group;
- $R_3$ represents a hydrogen atom, a halogen atom, such as a chlorine, bromine, iodine or fluorine atom, a $C_1$-$C_4$ alkyl radical, a $C_1$-$C_4$ monohydroxy-alkyl radical, a $C_1$-$C_4$ hydroxyalkoxy radical, a $C_1$-$C_4$ acetylaminoalkoxy radical, a $C_1$-$C_4$ mesylaminoalkoxy radical or a $C_1$-$C_4$ carbamoylaminoalkoxy radical;
- $R_4$ represents a hydrogen or halogen atom or a $C_1$-$C_4$ alkyl radical.

3. Composition according to Claim 2, **characterized in that** the para-phenylenediamines of formula (I) are chosen from para-phenylenediamine, paratoluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine,

4-amino-N,N-bis(β-hydroxyethyl)-3-methylaniline, 4-amino-3-chloro-N,N-bis(β-hydroxyethyl)aniline, 2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)amino-para-phenylenediamine and their addition salts with an acid.

**4.** Composition according to any one of the preceding claims, **characterized in that** the double bases are chosen from the compounds corresponding to the following formula (II) and their addition salts with an acid:

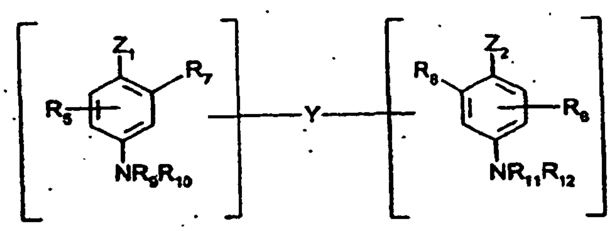

(II)

in which:

- $Z_1$ and $Z_2$, which are identical or different, represent a hydroxyl or $-NH_2$ radical which can be substituted by a $C_1$-$C_4$ alkyl radical or by a connecting arm Y;
- the connecting arm Y represents a linear or branched alkylene chain comprising from 1 to 14 carbon atoms which can be interrupted or terminated by one or more nitrogenous groups and/or by one or more heteroatoms, such as oxygen, sulphur or nitrogen atoms, and which is optionally substituted by one or more hydroxyl or $C_1$-$C_6$ alkoxy radicals;
- $R_5$ and $R_6$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a connecting arm Y;
- $R_7$, $R_8$, $R_9$, $R_{10}$, $R_{11}$ and $R_{12}$, which are identical or different, represent a hydrogen atom, a connecting arm Y or a $C_1$-$C_4$ alkyl radical;

it being understood that the compounds of formula (II) only comprise a single connecting arm Y per molecule.

**5.** Composition according to Claim 4, **characterized in that** the double bases of formula (II) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis (4-aminophenyl)tetramethylenediamine, N,N'-bis(4-methylaminophenyl)tetra-methylenediamine, N,N'-diethyl-N, N'-bis(4'-amino-3'-methylphenyl)ethylenediamine, 1,8-bis(2,5-diaminophenoxy)-3,5-dioxaoctane and their addition salts with an acid.

**6.** Composition according to any one of the preceding claims, **characterized in that** the para-aminophenols are chosen from the compounds corresponding to the following formula (III) and their addition salts with an acid:

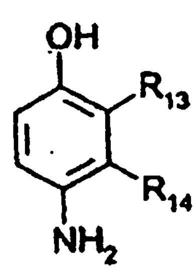

(III)

in which:

- $R_{13}$ represents a hydrogen or halogen atom, a $C_1$-$C_4$ alkyl radical, a $C_1$-$C_4$ monohydroxyalkyl radical, a ($C_1$-$C_4$) alkoxy($C_1$-$C_4$) alkyl radical, a $C_1$-$C_4$ aminoalkyl radical or a hydroxy($C_1$-$C_4$)alkylamino- ($C_1$-$C_4$)alkyl radical,
- $R_{14}$ represents a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical, a $C_1$-$C_4$ cyanoalkyl radical or a ($C_1$-$C_4$) alkoxy ($C_1$-$C_4$) alkyl radical,

it being understood that at least one of the $R_{13}$ or $R_{14}$ radicals represents a hydrogen atom.

**7.** Composition according to Claim 6, **characterized in that** the para-aminophenols are chosen from para-aminophenol, 4-amino-3-methylphenol, 4-amino-3-fluorophenol, 4-amino-3-(hydroxymethyl)phenol, 4-amino-2-methylphenol, 4-amino-2-(hydroxymethyl)-phenol, 4-amino-2-(methoxymethyl)phenol, 4-amino-2-(amino-methyl)phenol, 4-amino-2-[(β-hydroxyethyl)amino-methyl]phenol, 4-amino-2-fluorophenol and their addition salts with an acid.

**8.** Composition according to any one of the preceding claims, **characterized in that** the heterocyclic bases are chosen from pyridine derivatives, pyrimidine derivatives, pyrazole derivatives, pyrazolopyrimidine derivatives and their addition salts with an acid.

**9.** Composition according to any one of the preceding claims, **characterized in that** 2-chloro-6-methyl-3-aminophenol and/or the addition salt or salts with an acid represent from 0.0001 to 5% by weight of the total weight of the dyeing composition.

**10.** Composition according to Claim 9, **characterized in that** 2-chloro-6-methyl-3-aminophenol and/or the addition salt or salts with an acid represent from 0.005 to 3% by weight of the total weight of the dyeing composition.

**11.** Composition according to any one of the preceding claims, **characterized in that** the combined oxidation bases represent from 0.0005 to 12% by weight of the total weight of the dyeing composition.

**12.** Composition according to Claim 11, **characterized in that** the combined oxidation bases represent from 0.005 to 6% by weight of the total weight of the dyeing composition.

**13.** Composition according to any one of the preceding claims, **characterized in that** the addition salts with an acid are chosen from hydrochlorides, hydrobromides, sulphates and tartrates, lactates and acetates.

**14.** Composition according to any one of the preceding claims, **characterized in that** it exhibits a pH of between 3 and 12.

**15.** Composition according to any one of the preceding claims, **characterized in that** it is provided in the form of liquids, creams or gels.

**16.** Process for dyeing keratinous fibres and in particular human keratinous fibres, such as hair, **characterized in that** at least one dyeing composition as defined in any one of Claims 1 to 15 is applied to these fibres and **in that** the colour is developed at acidic, neutral or alkaline pH using an oxidizing agent which is added only at the time of use to the dyeing composition or which is present in an oxidizing composition applied simultaneously or sequentially in a separate fashion.

**17.** Process according to Claim 16, **characterized in that** the oxidizing agent present in the oxidizing composition is chosen from hydrogen peroxide, urea hydrogen peroxide, alkali metal bromates, persalts, such as perborates and persulphates, or peracids.

**18.** Dyeing multi-compartment device or kit with several compartments, a first compartment of which includes a dyeing composition as defined in any one of Claims 1 to 15 and a second compartment of which includes an oxidizing composition.